# EUROPÄISCHE PATENTSCHRIFT

(11) **EP 0 416 584 B1**
(45) Veröffentlichungstag und Bekanntmachung des Hinweises auf die Patenterteilung: **23.03.1994**
(21) Anmeldenummer: 90117092.8
(22) Anmeldetag: 05.09.1990
(51) Int. Cl.: C07D 251/28, B01J 2/10

(54) **Verfahren zur Verbesserung der Fliessfähigkeit von festem Cyanurchlorid**
Process to improve the flowability of solid cyanuric chloride
Procédé pour améliorer la fluidité de chlorure de cyanuryle solide

(30) Priorität: 07.09.1989 CH 3252/89
(43) Veröffentlichungstag der Anmeldung: 13.03.1991
(73) Patentinhaber: LONZA AG, CH-3945 Gampel/Wallis (CH)
(72) Erfinder: Skaria, Alexander, Dr., Stäfa (Zürich) (CH)
(74) Vertreter: Weinhold, Peter, Dr.

(56) Entgegenhaltungen:
- AT-B- 375 930
- DE-A- 2 834 543
- DE-B- 1 134 999
- DE-B- 2 412 262
- GB-A- 1 132 968
- KIRK-OTHMER: "Encyclopedia of chemical technology", 3. Auflage, Band 21, 1983,New York, Seiten 77-105
- CHEMICAL ABSTRACTS, vol. 69, no. 25, 16 Dezember 1968 Columbus, Ohio, USA Seite 10009; ref. no. 106756R

## Beschreibung

Die vorliegende Erfindung betrifft ein Verfahren zur Verbesserung der Fliessfähigkeit von feinkörnigem festem Cyanurchlorid.

Cyanurchlorid ist ein wichtiges Zwischenprodukt bei der Herstellung von Farbstoffen, optischen Aufhellern, Pflanzenschutzmitteln, pharmazeutischen Wirkstoffen sowie Textil-, Papier- und Kunststoffhilfsmitteln.

Technisch wird Cyanurchlorid durch Trimerisierung von Chlorcyan in der Gasphase hergestellt. Das bei der Trimerisierung anfallende gasförmige Cyanurchlorid wird entweder durch Einleiten in einen kalten Inertgasstrom direkt desublimiert oder zuerst verflüssigt und dann sprühkristallisiert. Dabei fällt es in Form eines sehr feinkörnigen Pulvers an. Es ist bekannt, dass dieses Pulver, insbesondere das durch Desublimation gewonnene, nur eine unbefriedigende Fliessfähigkeit besitzt.
Diese schlechte Fliessfähigkeit führt zu Schwierigkeiten bei der Lagerung, dem Transport, der Dosierung und der Weiterverarbeitung des Produkts. Bei der Lagerung neigt es zur Klumpen- und Brückenbildung, welche die Entnahme aus Silos erschweren, und beim Transport durch Rohrleitungen kommt es leicht zu Verstopfungen. Die Dosierung und beispielsweise das Auflösen in Lösungsmitteln bei der Weiterverarbeitung werden durch ungleichmässigen Materialfluss behindert.
Es ist daher notwendig und üblich, Massnahmen zur Verbesserung der Fliessfähigkeit des Cyanurchlorids durchzuführen. Zu diesem Zweck wurden bisher dem Produkt hochdisperse Kieselsäuren zugesetzt, die zum Beispiel unter der Bezeichnung Aerosil^{R} bekannt sind und auch bei zahlreichen anderen Stoffen als Fliesshilfsmittel eingesetzt werden. Diese Substanz ist zwar weitgehend inert, es ist jedoch für gewisse Anwendungen unerwünscht, dem Cyanurchlorid Fremdstoffe beizumischen.

Der vorliegenden Erfindung lag daher die Aufgabe zugrunde, ein Verfahren zur Verfügung zu stellen, welches gestattet, ohne grossen technischen Aufwand, insbesondere aber ohne störende Zusatzstoffe, die Fliesseigenschaften des feinkörnigen Cyanurchloridpulvers unter Beibehaltung seiner Reaktivität zu verbessern.

Erfindungsgemäss wird diese Aufgabe durch ein Verfahren nach Patentanspruch 1 gelöst.

Es wurde überraschenderweise gefunden, dass Cyanurchloridpulver, welches bei erhöhter Temperatur und unter Einwirkung genügend grosser Scherkräfte gerührt bzw. geknetet wurde, seine Korngrössenverteilung ändert und insbesondere seine Fliesseigenschaften erheblich verbessert. Dass eine derartige Behandlung zu einer Teilchenvergrösserung führen kann, ist an sich bekannt, so wird beispielsweise von C.E. Capes in Kirk-Othmer's Encyclopedia of Chemical Technology, 3. Aufl., Bd. 21, S. 77ff, auf S. 87f die Verwendung von Mischern verschiedener Bauart zur Agglomeration feinteiliger Materialien beschrieben. Bei diesen bekannten Anwendungen ist jedoch die Anwesenheit einer flüssigen Phase, sei es in Form eines aufgesprühten Bindemittels oder in Form von Feuchtigkeit des zu agglomerierenden Materials selbst, erforderlich, um feste Agglomerate zu erhalten, die bei mechanischer Belastung nicht sofort wieder zerfallen.
Das erfindungsgemässe Verfahren kommt dagegen völlig ohne Zusatz von Flüssigkeit oder Bindemittel aus und führt trotzdem zu druckfesten Körnern von Cyanurchlorid.
Das Cyanurchlorid wird erfindungsgemäss entweder direkt nach der Entnahme aus der Desublimierkammer bzw. der Sprühkristallisationskammer oder nach einer Zwischenlagerung in einem Kneter oder Mischer bei einer Temperatur von zweckmässig 20 bis 146°C, vorzugsweise 60 bis 120°C, umgewälzt. Die Knet- bzw. Mischvorrichtung ist vorzugsweise beheizbar, besonders bevorzugt sind beheizbare Mischer mit rotierenden Mischwerkzeugen, wie beispielsweise Pflugschar-, Leitschaufel- oder Paddelmischer. Die Wärmezufuhr kann sowohl durch Aussenbeheizung, etwa in Form eines Heizmantels, der von einem der üblichen Wärmeträger durchströmt wird oder elektrische Heizelemente enthält, als auch über entsprechend ausgebildete Knet- bzw. Mischerwerkzeuge oder durch eine Kombination beider Methoden erfolgen. Es ist auch möglich, die Wärmezufuhr ganz oder teilweise durch Einblasen eines erhitzten Inertgases in den Kneter bzw. Mischer bzw. das darin befindliche Cyanurchlorid zu bewirken. In diesem Fall werden die Abgase, welche zwangsläufig etwas verdampftes Cyanurchlorid enthalten, vorteilhaft in die Desublimationskammer zurückgeführt, um die Substanzverluste möglichst gering zu halten. Eine besonders rasche und effiziente Wärmezufuhr kann durch eine Kombination der beschriebenen Methoden erreicht werden. Die Dauer der Behandlung liegt vorzugsweise zwischen 10 Minuten und 10 Stunden und hängt von der Temperatur, der Intensität des Mischvorgangs und der gewünschten Korngrössenverteilung ab. Unter den bei den Ausführungsbeispielen gewählten Bedingungen haben sich Behandlungsdauern von 1 bis 2 Stunden als besonders vorteilhaft erwiesen.
Für die erzielte Kornstruktur spielen neben der thermischen Einwirkung in erster Linie die auftretenden Scherkräfte eine Rolle. Die Drehzahl der Knet- bzw. Mischelemente wird daher zweckmässig so gewählt, dass sich aus der Umlaufgeschwindigkeit und den Dimensionen der Scherspalte eine genügende Scherbeanspruchung des zu behandelnden Materials ergibt. Die Umlaufgeschwindigkeit der Knet- bzw. Mischelemente liegt vorzugsweise zwischen 0,1 und 10 m/s, besonders bevorzugt ist der Bereich zwischen 2 und 5 m/s.
Nach der Behandlung wird das Cyanurchlorid vorteilhaft zunächst auf 10 bis 50°C, vorzugsweise 20 bis 30°C, also etwa Raumtemperatur, abgekühlt. Die Abkühlung kann in derselben Vorrichtung wie die mechanische Behandlung erfolgen, es ist aber auch möglich, diese in einem separaten Mischer gleicher oder abweichender Bauart vorzunehmen. Da hierbei lediglich ein ausreichender Wärmeübergang gewährleistet sein muss, sind zu diesem Zweck auch Vorrichtungen verwendbar, in denen das Füllgut keiner wesentlichen Scherbeanspruchung unterliegt, wie beispielsweise Trommel- oder Freifallmischer. Die Wärmeabfuhr kann auf die gleiche Weise wie die Wärmezufuhr über die Wandungen und/oder die Mischelemente erfolgen, es ist aber auch möglich, durch Einbringen eines kalten oder kondensierten Gases wie beispielsweise Luft oder Stickstoff in flüssigem oder gasförmigem Zustand oder verflüssigten Kohlendioxids eine direkte Kühlung zu erzielen.
Nach der Abkühlung wird das Cyanurchlorid auf bekannte Weise ausgetragen und kann nun verpackt oder gelagert werden. Falls dies gewünscht wird, kann durch Vermischen mit einem bekannten Hilfsmittel wie beispielsweise dem oben erwähnten Aerosil^{R} eine weitere Verbesserung der Fliessfähigkeit erreicht werden.

Die nachfolgenden Beispiele verdeutlichen die Durchführung des erfindungsgemässen Verfahrens.

### Beispiele

Die Beispiele 1 bis 3 wurden mit desublimiertem Cyanurchlorid durchgeführt. Die Fliessfähigkeit wurde jeweils mit dem Jenike-Schertest bestimmt. Als Mass für die Fliessfähigkeit betrachtet man dabei das Verhältnis von Verfestigungsspannung σ₁ zu Schüttgutfestigkeit f_{c}. Dieses Verhältnis bezeichnet man als Jenike-Fliessfunktion FF_{c} (FF_{c} = σ₁/f_{c}). Das unbehandelte Cyanurchlorid weist typischerweise folgende Werte auf:
- ohne Fliessmittelzusatz :: FF_{c} = 1,8 - 2,0
- mit Fliessmittelzusatz :: FF_{c} = 4,0 - 6,0

### Beispiel 1

In einem beheizbaren Kneter mit 2 l Fassungsvermögen wurde 1 kg Cyanurchlorid mit einer mittleren Korngrösse von 11 µm bei 100°C mit 40 Umdrehungen/min (Umfangsgeschwindigkeit = 0,12 m/s) geknetet. Bereits nach einer Stunde war die mittlere Korngrösse auf 15 µm und der FF_{c}-Wert auf 14 angestiegen. Bei Fortsetzung der Behandlung nahm die mittlere Korngrösse stetig weiter zu und erreichte nach insgesamt 6 Stunden einen Wert von 40 µm.

### Beispiel 2

In einem beheizbaren Pflugscharmischer (Fabrikat LOEDIGE, Typ VT50) wurden 25 kg Cyanurchlorid bei einer Mischguttemperatur von 85°C und einer Umlaufgeschwindigkeit der Mischelemente von 2,5 m/s gemischt. Die mittlere Korngrösse erreichte ausgehend von einem Anfangswert von 11 µm nach 1 Stunde 15 µm und nach 2 Stunden 25 µm. Die Fliessfähigkeit war bereits nach einer Stunde erheblich verbessert, so dass nach Zugabe von Fliessmittel ein FF_{c}-Wert von 21 gemessen werden konnte.

### Beispiel 3

Es wurde wie im Beispiel 2 verfahren, jedoch mit 95°C Mischguttemperatur und einer Umlaufgeschwindigkeit von 3,14 m/s. Die mittlere Korngrösse stieg von 11 µm auf 18 µm (nach 1 Stunde) bzw. 25 µm (nach 2 Stunden).

Der FF_{c}-Wert betrug nach einer Stunde 5,5 (ohne Fliessmittelzusatz) bzw. 9,7 (mit Fliessmittelzusatz).

## Patentansprüche

1. Verfahren zur Verbesserung der Fliessfähigkeit von festem Cyanurchlorid, dadurch gekennzeichnet, dass man Cyanurchloridpulver in einem Kneter oder Mischer ohne Zusatz von Bindemittel bei einer Temperatur von 20 bis 146°C unter Einfluss von Scherung einer Teilchenagglomeration unterzieht.

2. Verfahren nach Patentanspruch 1, dadurch gekennzeichnet, dass die Teilchenagglomeration bei einer Temperatur von 60 bis 120°C erfolgt und das Produkt anschliessend auf Raumtemperatur abgekühlt wird

3. Verfahren nach Patentanspruch 2, dadurch gekennzeichnet, dass die Abkühlung indirekt über die Mischelemente und/oder die Wandung oder direkt durch Eintragen eines inerten Kühlmediums erfolgt.

4. Verfahren nach einem oder mehreren der Patentansprüche 1 bis 3, dadurch gekennzeichnet, dass die zum Erreichen der Mischtemperatur notwendige Wärmezufuhr ganz oder teilweise über die Mischelemente und/oder die Wandung erfolgt.

5. Verfahren nach einem oder mehreren der Patentansprüche 1 bis 4, dadurch gekennzeichnet, dass die zum Erreichen der Mischtemperatur erforderliche Wärmezufuhr ganz oder teilweise durch Einblasen eines heissen Inertgases in den Kneter oder Mischer erfolgt.

6. Verfahren nach einem oder mehreren der Patentansprüche 1 bis 5, dadurch gekennzeichnet, dass die Umlaufgeschwindigkeit der Knet- oder Mischelemente 0,1 bis 10 m/s, z.B. 2 bis 5 m/s, beträgt.

7. Verfahren nach einem oder mehreren der Patentansprüche 1 bis 6, dadurch gekennzeichnet, dass die Verweilzeit des Cyanurchlorids im Mischer oder Kneter 10 Minuten bis 10 Stunden, z.B. 1 bis 2 Stunden, beträgt.

8. Verfahren nach einem oder mehreren der Patentansprüche 1 bis 7, dadurch gekennzeichnet, dass die Teilchenagglomeration in einem beheizbaren Pflugscharmischer durchgeführt wird.

9. Verfahren nach einem oder mehreren der Patentansprüche 1 bis 8, dadurch gekennzeichnet, dass dem Cyanurchlorid zusätzlich hochdisperse Kieselsäure zugemischt wird.

10. Fliessfähiges Cyanurchlorid, erhalten durch das Verfahren nach einem oder mehreren der Patentansprüche 1 bis 9.

## Claims

1. A process for improving the flowability of solid cyanuric chloride, characterized in that cyanuric chloride powder in a kneader or mixer is subjected to particle agglomeration under the influence of shearing without addition of binder and at a temperature of from 20 to 146°C.

2. The process according to Claim 1, characterized in that particle agglomeration takes place at a temperature of from 60 to 120°C, the product being subsequently cooled down to room temperature.

3. The process according to Claim 2, characterized in that cooling is accomplished indirectly via the mixing elements and/or the walls, or directly by incorporation of an inert cooling medium.

4. The process according to one or more of Claims 1 to 3, characterized in that the heat required for reaching the mixing temperature is supplied in full or in part via the mixing elements and/or the walls.

5. The process according to one or more of Claims 1 to 4, characterized in that the heat required for reaching the mixing temperature is supplied in full or in part by blowing a hot inert gas into the kneader or mixer.

6. The process according to one or more of Claims 1 to 5, characterized in that the rotational speed of the kneading or mixing elements is from 0.1 to 10 m/s, e.g. from 2 to 5 m/s.

7. The process according to one or more of Claims 1 to 6, characterized in that the dwelling time of the cyanuric chloride in the mixer or kneader is from 10 minutes to 10 hours, e.g. from 1 to 2 hours.

8. The process according to one or more of Claims 1 to 7, characterized in that particle agglomeration is conducted in a heatable plow-type mixer.

9. The process according to one or more of Claims 1 to 8, characterized in that highly disperse silicic acid is additionally admixed with the cyanuric chloride.

10. Flowable cyanuric chloride, obtained by the process set forth in one or more of Claims 1 to 9.

## Revendications

1. Procédé pour améliorer la fluidité du chlorure de cyanuryle solide, caractérisé en ce que l'on soumet une poudre de chlorure de cyanuryle à une agglomération des particules sous l'influence d'un cisaillement dans un malaxeur ou un mélangeur sans addition de liant à une température de 20 à 146°C.

2. Procédé selon la revendication 1, caractérisé en ce que l'agglomération des particules est accomplie à une température de 60 à 120°C et le produit est ensuite refroidi à la température ambiante.

3. Procédé selon la revendication 2, caractérisé en ce que le refroidissement est accompli indirectement par l'intermédiaire des éléments de mélange et/ou de la paroi ou directement par introduction d'un agent réfrigérant inerte.

4. Procédé selon une ou plusieurs des revendications 1 à 3, caractérisé en ce que l'apport de chaleur nécessaire pour atteindre la température de mélange est accompli totalement ou en partie par l'intermédiaire des éléments de mélange et/ou de la paroi.

5. Procédé selon une ou plusieurs des revendications 1 à 4, caractérisé en ce que l'apport de chaleur nécessaire pour atteindre la température de mélange est accompli totalement ou en partie par insufflation d'un gaz inerte chaud dans le malaxeur ou le mélangeur.

6. Procédé selon une ou plusieurs des revendications 1 à 5, caractérisé en ce que la vitesse périphérique des éléments de malaxage ou de mélange est de 0,1 à 10 m/s, par exemple de 2 à 5 m/s.

7. Procédé selon une ou plusieurs des revendications 1 à 6, caractérisé en ce que le temps de séjour du chlorure de cyanuryle dans le malaxeur ou le mélangeur est de 10 minutes à 10 heures, par exemple de 1 à 2 heures.

8. Procédé selon une ou plusieurs des revendications 1 à 7, caractérisé en ce que l'agglomération des particules est accomplie dans un mélangeur à socs de charrue chauffable.

9. Procédé selon une ou plusieurs des revendications 1 à 8, caractérisé en ce qu'un acide silicique hautement dispersé est ajouté en outre au chlorure de cyanuryle.

10. Chlorure de cyanuryle fluide, obtenu par le procédé selon une ou plusieurs des revendications 1 à 9.
